(19) Europäisches Patentamt
European Patent Office
Office européen des brevets

(11) **EP 4 264 308 B1**

(12) **EUROPEAN PATENT SPECIFICATION**

(45) Date of publication and mention
of the grant of the patent:
**05.02.2025 Bulletin 2025/06**

(21) Application number: **21835335.7**

(22) Date of filing: **20.12.2021**

(51) International Patent Classification (IPC):
**G01R 33/563** (2006.01)   **G01R 33/56** (2006.01)
**G06N 3/04** (2023.01)   **A61B 6/00** (2024.01)
**G06N 3/08** (2023.01)   **A61B 5/055** (2006.01)
**A61B 6/03** (2006.01)

(52) Cooperative Patent Classification (CPC):
**G01R 33/56366; A61B 6/481; A61B 6/50;
A61B 6/507; A61B 6/5211; G01R 33/5601;
G01R 33/5608; G06N 3/045; G06N 3/049;
G06N 3/08;** A61B 5/055; A61B 6/032; A61B 6/037

(86) International application number:
**PCT/EP2021/086794**

(87) International publication number:
**WO 2022/129633 (23.06.2022 Gazette 2022/25)**

(54) **METHODS FOR TRAINING A CNN AND FOR PROCESSING AN INPUTTED PERFUSION SEQUENCE USING SAID CNN**

VERFAHREN ZUM TRAINIEREN EINES CNN UND ZUR VERARBEITUNG EINER EINGEGEBENEN PERFUSIONSSEQUENZ UNTER VERWENDUNG DES CNN

PROCÉDÉS PERMETTANT D'ENTRAÎNER UN CNN ET DE TRAITER UNE SÉQUENCE DE PERFUSION ENTRÉE EN UTILISANT LEDIT CNN

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **18.12.2020 EP 20306625**

(43) Date of publication of application:
**25.10.2023 Bulletin 2023/43**

(73) Proprietor: **Guerbet**
**93420 Villepinte (FR)**

(72) Inventors:
• **BONE, Alexandre**
**75009 PARIS (FR)**
• **VERON VIALARD, Julien**
**75007 PARIS (FR)**
• **ROBERT, Philippe**
**75010 PARIS (FR)**
• **ROHE, Marc-Michel**
**75011 PARIS (FR)**

(74) Representative: **Regimbeau**
**20, rue de Chazelles**
**75847 Paris Cedex 17 (FR)**

(56) References cited:
**CN-A- 110 223 285**

• **SANDFORT VEIT ET AL: "Reliable segmentation
of 2D cardiac magnetic resonance perfusion
image sequences using time as the 3rd
dimension", EUROPEAN RADIOLOGY,
SPRINGER BERLIN HEIDELBERG, BERLIN/
HEIDELBERG, vol. 31, no. 6, 27 November 2020
(2020-11-27), pages 3941 - 3950, XP037454995,
ISSN: 0938-7994, [retrieved on 20201127], DOI:
10.1007/S00330-020-07474-5**

- HESS ANDREAS ET AL: "Synthetic Perfusion Maps: Imaging Perfusion Deficits in DSC-MRI with Deep Learning", 26 January 2019, ADVANCES IN DATABASES AND INFORMATION SYSTEMS; [LECTURE NOTES IN COMPUTER SCIENCE; LECT.NOTES COMPUTER], SPRINGER INTERNATIONAL PUBLISHING, CHAM, PAGE(S) 447 - 455, ISBN: 978-3-319-10403-4, XP047502348

- KIM DAHUN ET AL: "Recurrent Temporal Aggregation Framework for Deep Video Inpainting", IEEE TRANSACTIONS ON PATTERN ANALYSIS AND MACHINE INTELLIGENCE, IEEE COMPUTER SOCIETY, USA, vol. 42, no. 5, 11 December 2019 (2019-12-11), pages 1038 - 1052, XP011780974, ISSN: 0162-8828, [retrieved on 20200401], DOI: 10.1109/TPAMI.2019.2958083

**Description**

FIELD OF THE INVENTION

The field of this invention is that of machine learning.

More particularly, the invention relates to methods for training a convolutional neural network for processing perfusion sequences, and for using such convolutional neural network, in particular for generating quantitative maps of perfusion parameters.

BACKGROUND OF THE INVENTION

Perfusion imaging, and in particular perfusion MRI, is an advanced biomedical imaging technique that allows to visualize and quantify the blood supply of an organ, such as the brain or the heart.

Perfusion MRI is widely used in clinical practice, notably in neuroimaging for the initial diagnosis and treatment planning of stroke and glioma.

There are 3 main techniques for perfusion MRI:

- Dynamic susceptibility contrast (DSC);
- Dynamic contrast enhanced (DCE); and
- Arterial spin labelling (ASL).

DSC and DCE, respectively leveraging T2 and T1 effects, are the two most common techniques. In both cases, a gadolinium-based contrast agent (GBCA) is injected intravenously to the patient and rapid repeated imaging is performed in order to obtain a temporal sequence of 3D images, referred to as a "raw" perfusion sequence. ASL (magnetic labeling of arterial blood below the imaging slab) interestingly does not rely on the injection of any GBCA, but is still significantly less widespread in clinical practice.

Quantitative maps of relevant perfusion parameters, such as the cerebral blood volume (CBV) or the cerebral blood flow (CBF), are usually extracted from the raw perfusion sequences before analysis by the radiologist.

Many different perfusion post-processing software solutions are commercially available and rely on microvascular models that are fitted in a voxel-wise fashion. The estimation of quantitative perfusion maps is classically framed as a deconvolution problem, whose closure relies on the preliminary estimation of the so-called arterial input function (AIF) by manually or semi-automatically delineating a large artery in the raw perfusion sequence. Solvers are predominately based on singular value decomposition (SVD) or later variations such as the circular SVD (oSVD).

However, these known solutions exhibit serious drawbacks:

1. The generation of the results may be very slow, and require up to several hours. This is not acceptable in the clinical scenario of stroke, where the time pressure is maximal.

2. Raw perfusion sequences may exhibit high levels of noise, and in turn low signal-to-noise ratios (SNR). As deconvolution is an ill-posed problem particularly sensitive to noise, even if a whole taxonomy of different regularization techniques has been proposed, the robust and repeatable estimation of perfusion quantitative maps is notoriously difficult.

3. In first-pass perfusion methods such as DSC, the observed signal is proportional to the injected quantity of GBCA, thus a solution to have "less degraded" results is to increase this injected quantity. However, based on precautionary considerations, recent clinical guidelines suggest using the minimum dosage that achieves a sufficient contrast enhancement. GBCA usage should therefore be as parsimonious as possible. Alternatively, or in addition, it would be interesting to use contrast agent with lower concentration (without increasing the injected quantity) so as to enable higher T1 relaxivity.

4. Mandatory input from the user should be avoided (e.g. no large vessel to segment or AIF function to choose) and the processing should be fully automatic, because classical perfusion post-processing solutions are known to produce highly variable results when the user changes. Furthermore, this manual input is cumbersome to the user.

The document Andreas Hess, Raphael Meier, Johannes Kaesmacher, Simon Jung, Fabien Scalzo, David Liebeskind, Roland Wiest, and Richard McKinley, "Synthetic perfusion maps: imaging perfusion deficits in dsc-mri with deep learning," in International MICCAI brain lesion workshop. Springer, 2018, pp. 447-455 and WO2017/192629 were the first to propose using a machine learning approach in order to imitate and reproduce classical software solutions, with the claimed advantages of being fully-automatic, faster, and more robust to noise. However, such solution did not turn out to be satisfactory. A similar approach for processing perfusion images by a convolutional neural network having an U-net architecture is known from prior art document CN110223285A.

There is consequently still a need for a new processing method of perfusion sequences that would be fully automatic, very fast (typically results in less than a second), and highly robust (to low SNR ratios).

SUMMARY OF THE INVENTION

For these purposes, the present invention provides according to a first aspect a method for processing an inputted perfusion sequence, by means of a convolutional neural network, CNN, the method being characterized in that it comprises the implementation, by a data processor of a second server, of steps of:

(b) extracting, using an encoder branch of the CNN, a plurality of initial n+1-dimensional features maps representative of the inputted perfusion sequence at different scales, n≥3, said CNN further comprising a decoder branch and skip connections between the encoder branch and the decoder branch, each skip connection projecting the initial n+1-dimensional features maps into initial n-dimensional feature maps;

(c) generating, using said decoder branch of the CNN, a plurality of enriched n-dimensional feature maps also representative of the inputted perfusion sequence at different scales, each enriched n-dimensional feature map incorporating the information from the initial n-dimensional feature maps of smaller or equal scale;

(d) generating at least one quantitative map of the inputted perfusion sequence from the largest-scale enriched n-dimensional feature maps.

Preferred but non limiting features of the present invention are as it follows:

For each enriched n-dimensional feature map, an initial n-dimensional feature map of the same scale is provided from the encoder branch to the decoder branch - via a dedicated skip connection.

At step (c), the smallest-scale enriched n-dimensional feature map is generated from the smallest-scale initial n+1-dimensional feature map, and each other enriched n-dimensional feature map is generated from the initial n-dimensional feature map of the same scale and a smaller-scale enriched n-dimensional feature map.

The method comprises a previous step (a) of obtaining the perfusion sequence by stacking a plurality of successive images of a perfusion.

Said successive images are acquired by a medical imaging device connected to the second server;

Said medical imaging device is a Magnetic Resonance Imaging, MRI, scanner, and the perfusion sequence is Dynamic susceptibility Contrast, DSC, or a Dynamic Contrast Enhanced, DCE, perfusion sequence; said quantitative map being preferably a map of parameter chosen among a cerebral blood volume, CBV, a cerebral blood flow, CBF, a mean transit time, MTT, and a k-trans.

Step (a) comprises pre-processing said successive images so as to improve quality of the perfusion sequence.

Step (a) comprises extracting patches of a predetermined size from the perfusion sequence, steps (b) to (d) being performed for each extracted patch.

Said CNN is fully convolutional.

The encoder branch of the CNN comprises n-dimensional convolution layers applying n-dimensional filters (in particular 3D convolution layers applying tridimensional filters), and the decoder branch of the CNN comprises n-1-dimensional convolution layers applying n-1-dimensional filters (in particular standard convolution layers applying bidimensional filters).

The perfusion sequence presents at least two spatial dimensions and one temporal dimension; the at least one quantitative map only presents said spatial dimensions; the initial n+1-dimensional feature maps present said spatial and temporal dimensional dimensions and as n+1-th dimension a semantic depth; and said initial and enriched n-dimensional feature maps present said spatial dimensions and as n-th dimension said semantic depth.

The number of said spatial dimensions is n-1, and preferably n=3.

Said skip connections perform a temporal pooling operation.

According to a second aspect, the invention provides a method for training a convolution neural network, CNN, for processing an inputted perfusion sequence;

the method being characterized in that it comprises the implementation, by a data processor of a first server, for each of a plurality of training perfusion sequence from a base of training perfusion sequences each associated to an expected quantitative map of the perfusion, of steps of:

(B) extracting, using an encoder branch of the CNN, a plurality of initial n+1-dimensional features maps representative of the training perfusion sequence at different scales, n≥3, said CNN further comprising a decoder branch and skip connections between the encoder branch and the decoder branch, each skip connection projecting the initial n+1-dimensional features maps into initial n-dimensional feature maps;

(C) generating, using said decoder branch of the CNN, a plurality of enriched n-dimensional feature maps also representative of the training perfusion sequence at different scales, each enriched n-dimensional feature map incorporating the information from the initial n-dimensional feature maps of smaller or equal scale;

(D) generating at least one candidate quantitative map of the perfusion sequence from the largest-scale enriched n-dimensional feature map, and minimizing a distance with the expected quantitative map of the perfusion.

Preferred but non limiting features of the present invention are as it follows:

The method previously comprises generating at least one degraded version of at least one original training perfusion sequence of the training base, associating to said degraded version the expected quantitative map of the perfusion associated with the original training perfusion sequence, and enriching the training base by adding said degraded version.

Said original training perfusion sequence is associated to a contrast product dose, said degraded version of the original training perfusion sequence simulating a lower contrast product dose.

The degraded version of the original training perfusion sequence simulating a lower contrast product dose is generated by calculating, for each voxel of the original training perfusion sequence, from the temporal signal $S(t)$ of said voxel a degraded temporal signal $S_d(t)$ using the formula $S_d(t) = S(t) - (1 - d) \cdot [\overline{S(t)} - S(0)]$, wherein $\overline{S(t)}$ is a local average of the temporal signal $S(t)$, and d is a dose reduction factor.

According to a third aspect the invention provides a computer program product comprising code instructions to execute a method according to the first aspect for training a convolution neural network CNN, or according to the second aspect for processing an inputted perfusion sequence.

BRIEF DESCRIPTION OF THE DRAWINGS

The above and other objects, features and advantages of this invention will be apparent in the following detailed description of an illustrative embodiment thereof, which is to be read in connection with the accompanying drawings wherein:

- figure 1 illustrates an example of architecture in which the methods according to the invention is performed;
- figure 2 illustrates an embodiment of the methods according to the invention;
- figure 3a represents a known U-Net architecture;
- figure 3b represents an example of CNN architecture for use in the methods according to the invention;
- figure 4a illustrates an artificial degradation feature used in a preferred embodiment of the methods according to the invention;
- figure 4b represents corresponding quantitative maps of the example perfusion sequences represented in figure 4a.

DETAILED DESCRIPTION OF A PREFERRED EMBODIMENT

*Architecture*

Two complementary aspects of the present invention are proposed:

- a method for training a Convolutional Neural Network (CNN) for processing an inputted perfusion sequence;
- a method for processing an inputted perfusion sequence using a CNN, advantageously trained according to the previous method.

By perfusion sequence, it is meant a stack of a plurality of images successively (the sequence is a "temporal" sequence) depicting "perfusion", i.e. passage of fluid through the lymphatic system or blood vessels to an organ or a tissue, for a person or an animal. In other words, the images constituting said perfusion sequence are images of a given body part to be monitored, depicting passage of a fluid within said body part. As it will be explained, the images could be either bidimensional or tridimensional.

In the following description, we will take the preferred example of blood perfusion of the brain, i.e. said fluid is the blood and said body part is the brain.

The images of the perfusion sequence are either directly acquired, or derived from images directly acquired, by a medical imaging device of the scanner type. In particular, in the following description, the sequence of images as acquired will be referred to as "raw" perfusion sequence, and the present method can take as input the raw perfusion sequence or an "improved" perfusion sequence having underwent a "pre-processing" in order to improve the quality of the sequence and/or to reduce its dimensionality, see below.

Said perfusion may be:

- CT (Computed Tomography) perfusion → the medical imaging device is an X-ray scanner;
- MRI (Magnetic Resonance Imaging) perfusion, and especially a DSC (Dynamic susceptibility Contrast) or a DCE (Dynamic Contrast Enhanced) perfusion -+ the medical imaging device is an MRI scanner;
- NM (Nuclear Medicine) perfusion -> the medical imaging device is a PET (positron emission tomography) scanner;
- Etc.

The acquisition of a said images may involve the injection of a contrast agent such as gadolinium (CBCA) for MRI or radioactive isotopes (for instance for the DSC/DCE perfusion).

Note that the present invention will not be limited to any type of input (as long as the images of the sequence represent the evolution of perfusion of a body part over time) and any origin (the perfusion sequence may simply be obtained from a database).

In any case, the inputted perfusion sequence are mathematically n-dimensional objects, with n≥3 (hence at least tridimensional objects, but possibly quadridimensional or even pentadimensional objects as it will be shown), having m spatial dimensions, with n>m≥2 (and preferably m=n-1), and one temporal dimension (according to which the images are stacked).

Generally, we have n=3 and m=2, which corresponds to tridimensional inputted perfusion sequence being temporal sequences of bidimensional images, hence "2+1D" objects.

Note that the raw perfusion sequences are actually often quadridimensional objects, having three spatial dimensions (said "images" of the sequences are actually 3D tomograms, i.e. volumes constituted of stacks of bidimensional images as "slices" of the tomogram according to a third spatial dimension - in other words, we have 2+1 spatial dimensions) and one temporal dimension (according to which the tomograms are further stacked).

In such a case, the inputted perfusion sequences may keep the structure of the quadridimensional raw perfusion sequences and thus we have n=4 and m=3, which corresponds to quadridimensional inputted perfusion sequence being temporal sequences of tridimensional images, hence "3+1D" objects. Alternatively, as it be shown, convolutional neural networks usually preferably deal with bidimensional images, so that a quadridimensional raw perfusion sequence can be processed slice by slice, i.e. as a collection of tridimensional inputted perfusion sequences as temporal sequences of all the bidimensional images of a given cross section in the tomograph.

Alternatively, or in addition, the raw perfusion sequence may be a "batch" of stacked independent complete 3D/4D perfusion sequences (if for instance the body part has been scanned several times in a row), hence a possible supplemental batch dimension (i.e. m=n-2), leading up to pentadimensional inputted perfusion sequences (n=5 and m=3). Again, as convolutional neural networks usually preferably deal with bidimensional images, such a batch penta/quadridimensional raw perfusion sequence can be processed independent sequence by independent sequence, i.e. as a collection of quadri/tridimensional inputted perfusion sequences, and possibly collections of collections of tridimensional inputted perfusion sequences.

Therefore, in the following description we will discuss the preferred example of a single tridimensional inputted perfusion sequence being processed, possibly as a part of a penta/quadridimensional raw perfusion sequence.

The images are numbered from 1 to T, T being for example equal to 120, for example one image per second for a duration of 2 minutes. In other words, a generic image of a perfusion sequence will be designated by a temporal coordinate *t*, with $t \in [\![1; T]\!]$, of the perfusion sequence. Each image is a matrix of pixels/voxels of a given size.

By processing a perfusion sequence, it is in particular meant the generation of at least one quantitative map of the perfusion, i.e. an image illustrating the value of a relevant parameter (i.e. a parameter characterizing said perfusion) over the body part to be monitored.

For example, in the case of DSC perfusion for brain imaging, said relevant parameter could be a cerebral blood volume (CBV), a cerebral blood flow (CBF) or a Mean transit time (MTT - calculated by dividing CBV by CBF), and the quantitative map is a CBV/CBF/MTT map of the brain.

As another example, in the case of DCE perfusion, the most commonly relevant parameter is k-trans (capillarity permeability), but other parameters such as the fractional volume of extravascular-extracellular space or the fractional volume of the plasma space can be chosen.

Note that the present processing is a "post-processing" if the perfusion sequence has already undergone pre-processing for quality improvement.

By contrast with the inputted perfusion sequence, the outputted quantitative maps are mathematically n-1-dimensional objects having only the m spatial dimensions, hence "2D" objects equivalent to images if m=2. Note that if m=3 and a quadridimensional raw perfusion sequence is processed slice by slice as a collection of tridimensional inputted perfusion sequences (n=2), i.e. the CNN is applied independently to each slice of the perfusion sequence, are generated as many quantitative maps slices that can be assembled so as to reconstitute complete tridimensional quantitative maps. In other words, the tridimensional quantitative map is obtained slice by slice (as a bidimensional quantitative map is obtained for each tridimensional inputted perfusion sequence corresponding to a slice).

Advantageously, perfusion sequences are "patched", i.e. patches are extracted from the tridimensional perfusion sequences, meaning they are divided into smaller and calibrated spatial pieces, for example "strips" of size 32x256, and the CNN is applied independently to each perfusion sequence patch so to generate as many quantitative maps patches (i.e. the input of the CNN may be a 3D patch of size 32x256x120 and the output may be a 2D patch of size 32x256) that can be assembled so as to reconstitute complete bidimensional quantitative maps (that may be assembled so as to reconstitute complete tridimensional quantitative maps).

Extracting patches allows a lighter CNN and allows operating on any size of perfusion sequence, but the invention is not limited to this embodiment, and the whole perfusion sequence could be processed.

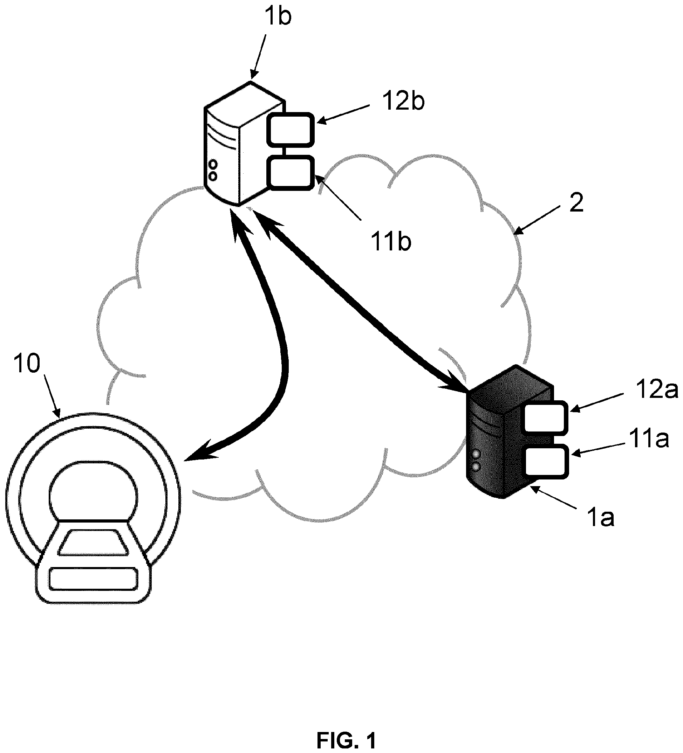

The above-mentioned methods are implemented within an architecture such as illustrated in **Figure** 1, by means of a first and/or second server 1a, 1b. The first server 1a is the training server (implementing the training method) and the second server 1b is a processing server (implementing the processing method). It is fully possible that these two servers may be merged.

Each of these servers 1a, 1b is typically remote computer equipment connected to an extended network 2 such as the Internet for data exchange. Each one comprises data processing means 11a, 11b of processor type (in particular the data processor 11a of the first server 1a have strong computing power, since learning is long and complex compared with ordinary use of the trained CNN), and optionally storage means 12a, 12b such as a computer memory e.g. a hard disk. The second server 1b may be connected to one or more medical imaging devices 10 as client equipment, for providing new perfusion sequences to be processed.

The memory 12a of the first server 1a stores a training database i.e. a set of already processed perfusion sequences referred to as training perfusion sequences (as opposed to so-called inputted perfusion sequence that precisely is sought to be processed in order to obtain the corresponding quantitative maps). As explained, the data is here constituted of perfusion sequences (in particular patched), the training examples being perfusion sequences associated with the corresponding quantitative maps as "expected results" typically previously computed with a reference perfusion sequence processing algorithm (software or machine learning approach).

*Obtaining the perfusion sequence*

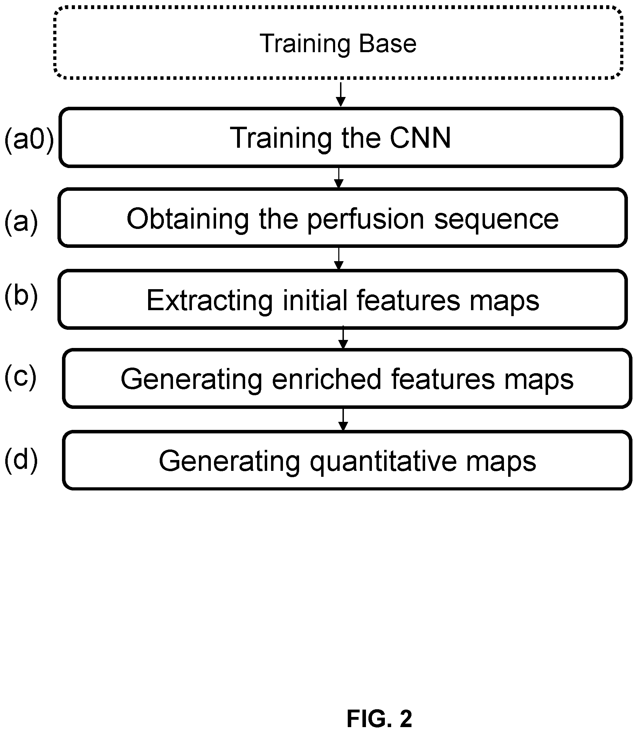

Preferably, as represented by **figure 2,** the method for processing an inputted perfusions sequence starts with a step (a) of obtaining said perfusion sequence to be processed. Indeed, as already explained, perfusion sequences are n-dimensional objects with m spatial dimensions and one temporal dimension, while a collection of m-dimensional images is produced by medical imaging devices, with $n \geq 3$, $n > m \geq 2$ (and preferably m=n-1, and most preferably n=3 and m=2).

Consequently, step (a) typically comprises stacking the plurality of successive images of a perfusion so as to generate the inputted perfusion sequence as a n-dimensional object, possibly after obtaining said images from a medical imaging device 10 (which may involve actually acquiring the images using the medical imaging device 10). Note that the inputted perfusion sequence may also be directly retrieved from a database.

Step (a) may also comprise pre-processing the perfusion sequence, in particular so as to generate an "improved" perfusion sequence (possibly with reduced dimensionality) from a "raw" prefusion sequence (which is the stack of the images as acquired from the medical imaging device 10). Note that the images may be pre-processed individually then stacked, or the opposite.

In a known fashion, pre-processing images of a raw perfusion sequence in step (a) may comprise one or more of:

- Filtering (for instance N4 bias correction);
- Signal normalization;
- Data augmentation (for instance rotation, flip etc.)
- Association with complementary sequences such as other MRI sequences not focusing on perfusion (sequences of T1-weighted images, post-contrast T1-weighted images, T2-weighted images, Flair images, diffusion maps, etc.)

Alternatively, or in addition, step (a) may also comprise extracting patches from the raw/improved perfusion sequences thanks to a "image-to-patch" generator that maps the whole image surface with the multiple patches and which is able to work as a "patch-to-image" generator to reconstitute an image. As already explained, patches are still 3D objects.

Note that this step (a) may be implemented by the data processor 11b of the second server 1b, or directly by the medical imaging device 10 if it has such computing capabilities.

*CNN*

The present invention introduces a new CNN architecture referred to as "spatio-temporal U-Net", or simply stU-Net, designed to very efficiently predict perfusion quantitative maps from inputted perfusion sequences, in particular with higher performances than the CNN proposed in the documents WO2017/192629 and Andreas Hess, Raphael Meier, Johannes Kaesmacher, Simon Jung, Fabien Scalzo, David Liebeskind, Roland Wiest, and Richard McKinley, "Synthetic perfusion maps: imaging perfusion deficits in dsc-mri with deep learning," in International MICCAI brain lesion workshop.

Springer, 2018, pp. 447-455., which by referred to as "Hess" CNN in the tests below.

The base U-Net, described in the document Olaf Ronneberger, Philipp Fischer, and Thomas Brox, "U-net: Convolutional networks for biomedical image segmentation," in International Conference on Medical image computing and computer-assisted intervention. Springer, 2015, pp. 234-241, is a well-known CNN for a very different purpose which is semantic segmentation of images.

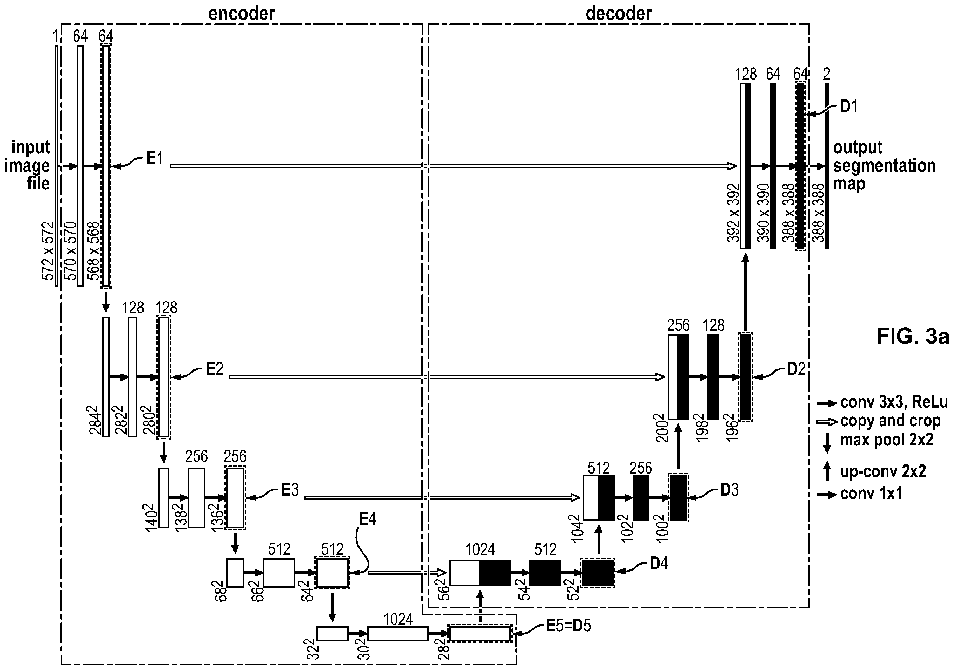

With reference to **figure 3a**, U-Net is a neural network of the encoder-decoder type: it comprises an encoder branch (or "contracting path") that maps the inputted image into a high-level representation and then a decoder branch (or "expanding path") generating the output image (the segmentation mask) from the high-level representation.

U-Net further comprises skip (or "lateral") connections between the encoder branch and decoder branch.

The encoder branch, acts as a backbone, and can be seen as a conventional feature extraction network that can be of many types, and in particular a conventional CNN, preferably a fully convolutional neural network (direct succession of blocks of convolution layers and non-linear layers such as ReLU). The encoder branch extracts from the input image a plurality of initial feature maps E1, E2, E3, E4, E5 representative of the input image at different scales. More precisely, the backbone consists of a plurality of successive convolution blocks, such that the first block produces a first initial feature map E2 from the input image, then the second block produces a second initial feature map E2 from the first initial feature map E1, etc.

It is conventionally understood for convolutional neural networks that the scale is smaller with each successive map (in other words the resolution decreases, the feature map becomes "smaller" and therefore less detailed), but of greater semantic depth, since increasingly high-level structures of the image have been captured. Specifically, initial feature maps have increasing numbers of channels as their spatial size decreases.

In practice, a pooling layer is placed between two blocks to decrease the scale by a factor of 2 (typically 2x2 max pooling operation with stride 2 for down sampling), and from one block to another the number of filters of the convolution layers used (generally 3x3 convolutions) is increased (and preferably doubled).

In the 5-level standard U-Net there is for example successive channel numbers of 64, 128, 256, 512 and 1024, and successive map spatial sizes (for a 572x572 input image) of 568x568, 280x280, 136x136, 64x64 and 28x28 (The cropping is necessary due to the loss of border pixels in every convolution).

The feature maps E1, E2, E3, E4, E5 obtained by the encoder branch are said to be initial because they will be reprocessed by the decoder branch. Indeed, as explained, "low-level" maps have a higher spatial resolution but a shallow semantic depth. The decoder branch aims to increase their semantic depth by incorporating the information from the "high-level" maps.

Thus, said decoder branch of the CNN has the symmetrical architecture of the encoder branch, as it generates, from the initial feature maps E1, E2, E3, E4, E5, a plurality of enriched feature maps D1, D2, D3, D4, D5 that are again representative of the input image at different scales, but they incorporate the information from the initial feature maps of smaller or equal scale while reducing the number of channels.

In other words, the decoder branch also consists of a plurality of successive convolution blocks but in opposite order, such that the first block produces the first enriched feature map D1 (from which the output image may be directly generated) from the second enriched feature map D2 and the first initial feature map E1, after the second block produces the second enriched feature map D2 from the third enriched feature map D3 and the second initial feature map E2, etc. The decoder branch is also preferably a fully convolutional CNN (direct succession of blocks of convolution layers and non-linear layers such as ReLU),

In more details, each i-th enriched map Di has the scale of the corresponding i-th initial map Ei (i.e. sensibly same spatial size) but incorporates the information of all j-th maps Di, for each j≥i. In practice, each i-th enriched map Di is generated according to the corresponding i-th initial map Ei and/or the next (i+1-th) enriched map Di+1, hence the "contracting and expansive" nature of the branches (i.e. "U" shaped): the initial maps are obtained in ascending order and then the enriched maps are obtained in descending order.

Indeed, the maximum semantic level is obtained at the "smallest-scale" map, and from there each map is enriched on the way back down again with the information of the already enriched maps. The skip connections between the encoder branch and the decoder branch provide the decoder branch with the various initial maps.

Typically, the generation of an enriched map Di based on the corresponding initial map Ei and the smaller-scale enriched map Di+1 comprises rescaling of the enriched map Di+1, typically doubling the scale (if there has been halving of scale in the encoder branch), i.e. up sampling of the enriched feature map with by a 2x2 convolution ("up-convolution") that halves the number of feature channels, then concatenation with the corresponding initial map Ei (cropped of necessary, both maps are now sensibly the same scale) to double again the number of channels, and from one block to another the number of filters of the convolution layers used (generally 3x3 convolutions) is again decreased (and preferably further halved).

For example, in the U-Net of figure 3a:

- The smallest-scale enriched map D5 (size 28x28x1024) is directly the smallest-scale initial map E5 (since the latter

8

already has the maximum semantic depth);

- The second smallest-scale enriched map D4 (size 52x52x512) is generated from the second smallest-scale initial map E4 (size 64x64x512) provided via the skip connection and the smallest-scale enriched map D5 (size 28x28x1024), by up-convolution of D5 (doubling its scale but halving its depth → resulting size (28x2)x(28x2) x(1024/2)=56x56x512) then concatenation of cropped initial map E4 (resulting size 56x56x512) with the up-convoluted D5 (resulting size 56x56x(512+512)=56x56x1024), from which the convolution block generates D4 (number of convolution filters halved, resulting size 52x52x512). Consequently, the information of the enriched map D5 has been added to the initial map E4;
- The third smallest-scale enriched map D3 (size 100x100x256) is generated from the third smallest-scale initial map E3 (size 136x136x256) provided via the skip connection and the second smallest-scale enriched map D4 (size 52x52x512), by up-convolution of D4 (doubling its scale but halving its depth → resulting size (52x2)x(52x2)x(512/2) =104x104x256) then concatenation of cropped initial map E4 (resulting size 104x104x256) with the up-convoluted D4 (resulting size 104x104x(256+256)=104x104x512), from which the convolution block generates D3 (number of convolution filters halved, resulting size 100x100x256). Consequently, the information of the enriched map D4 has been added to the initial map E3;
- Etc.

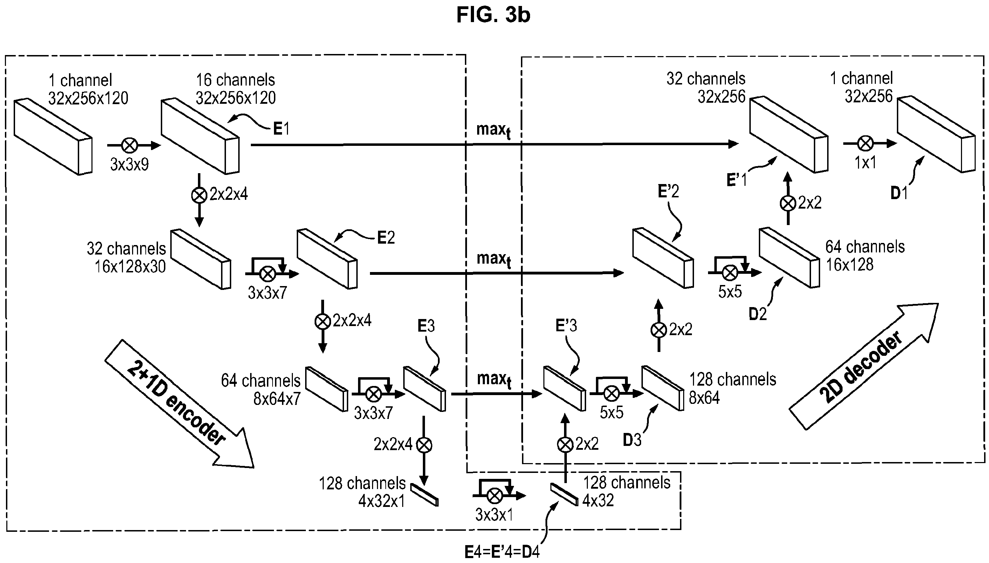

As represented by the **figure 3b,** the stU-Net type CNN used in the methods according to the invention still have an encoder branch, a decoder branch, and skip connections, but it notably differs from U-Net or other known CNN in that the encoder and the decoder branches do not have the same number of dimensions, leading to an asymmetrical architecture.

In particular we have a m-dimensional (preferably 2D) decoder branch with a m+1-dimensional (preferably 3D) encoder branch able to exploit the intrinsic spatio-temporal regularities of perfusion data using convolution filters that jointly act on the spatial and temporal domains (it can be seen in the case of tridimensional inputted perfusion sequences as a "2+1D" encoder).

Note that a "3D U-Net" called V-Net, see Fausto Milletari, Nassir Navab, and Seyed-Ahmad Ahmadi, "V-net: Fully convolutional neural networks for volumetric medical image segmentation," in 2016 fourth international conference on 3D vision (3DV). IEEE, 2016, pp. 565-571, is already known, but such CNN does not anticipate at all stU-Net because:

- It stays symmetrical, with both 3D encoder and 3D decoder;
- V-Net has 3 spatial dimensions (it works on volumetric data), and therefore it does not handle spatio-temporal information;
- It presents a key architectural difference in the skip connections, see below.

*Encoding and decoding using stU-Net*

Steps (b) to (d), implemented by the data processor 11b of the second server 1b, correspond to the application of the stU-Net type CNN to the inputted perfusion sequence.

The present CNN still successively comprises an encoder branch, a decoder branch, and skip connections between the encoder branch and the decoder branch.

In a step (b), the encoder branch of the CNN extracts a plurality of initial n+1-dimensional features maps E1, E2, E3, E4 (at least two, advantageously at least three, more advantageously less than five, optimally four) representative of the inputted perfusion sequence at different scales.

Indeed, as it is a m+1-dimensional encoder, the corresponding feature maps are at least m+2-dimensional, and m+3-dimensional if there is a batch dimension. In the first case m=n-1 so m+2=n+1, and in the second case m=n-2 so m+3=n+1. The perfusion sequence presents n dimensions including m spatial dimensions (dimensions of each image) one temporal sequence (dimension according to which the images are stacked) and possibly one batch dimension, therefore the initial n+1-dimensional feature maps advantageously present as first n dimension said spatial, temporal and batch dimensions, and as n+1-th dimension a semantic depth (number of channels). If n=3 (most common case of tridimensional inputted perfusion sequence), then we have quadridimensional features maps, if n=4 (m=3 or there is a batch dimension), then we have pentadimensional features maps, if n=5 (m=3 and there is a batch dimension), then we have hexadimensional features maps.

Note that the encoder branch can be simply fed with the n-dimensional inputted perfusion sequence patch by considering it as a "mono-channel" n+1-dimensional object, see below.

Like U-Net/V-Net, such encoder branch advantageously consists of a plurality of successive convolution blocks, such that the first block produces a first initial feature map E2 from the input image, then the second block produces a second initial feature map E2 from the first initial feature map E1, etc., but with n-dimensional convolution layers applying n-dimensional filters in particular at least 3D convolution layers applying tridimensional filters, for instance spatio-temporal convolutions, using anisotropic kernels of size 3x3x7 (or 3x3x1 for the last block, see below) with stride 1.

Between two blocks, the scale may be reduced, halved, or even divided by four for instance by "down-convolution" using size 2x2x4 with stride 4 for down sampling (both spatial sizes are halved, and temporal size is divided by four, while depth is doubled), but pooling operation may alternatively be used.

Note that alternatively the skilled person could use any known architecture of n-dimensional encoder, for instance the V-Net 3D encoder.

In the example of figure 3b:

- The encoder is fed with perfusion sequence patch of size 32x256x120 which is handled as a "mono-channel" 4D object of size 32x256x120x1;
- A (16-filters) first convolution block generates a first initial quadridimensional features map E1 of size 32x256x120x16,
- The first initial quadridimensional features map E1 is down sampled to a size of (32/2)x(256/2)x(120/4)x(16x2) =16x128x30x32, then a (32-filters) second convolution block generates a second initial quadridimensional features map E2 of size 16x128x30x32;
- The second initial quadridimensional features map E2 is down sampled to a size of (16/2)x(128/2)x(30/4)x(32x2) =8x64x7x64, then a (64-filters) third convolution block generates a third initial quadridimensional features map E3 of size 8x64x7x64;
- The third initial quadridimensional features map E3 is down sampled to a size of (8/2)x(64/2)x(7/4)x(64x2) =4x32x1x128, then a (128-filters) fourth convolution block (using kernels of size 3x3x1 because there is only one remaining temporal channel) generates the fourth initial quadridimensional features map E4 of size 4x32x1x128.

In a following step (c), the decoder branch generates a plurality of enriched n-dimensional feature maps D1, D2, D3, D4 also representative of the inputted perfusion sequence at different scales.

The difficulty is now that there is not a dimensional consistency between the encoder and the decoder: said enriched n-dimensional feature maps only present the m spatial dimensions, the possible batch dimension, and as n-th dimension said semantic depth, but not the temporal dimension.

Nevertheless, we would like to have each enriched n-dimensional feature map D1, D2, D3, D4 incorporating the information from the initial n+1-dimensional feature maps E1, E2, E3, E4 of smaller or equal scale.

To solve this problem, the present invention astutely proposes to have the skip connection projecting the initial n+1-dimensional features maps E1, E2, E3, E4 into initial n-dimensional feature maps E'1, E'2, E'3, E'4, instead of simply providing the initial n+1-dimensional features maps E1, E2, E3, E4 to the decoder branch. In other words, the decoder is fed with initial feature maps with a reduced number of dimensions, and typically for each enriched n-dimensional feature map D1, D2, D3, D4, an initial n-dimensional feature map E'1, E'2, E'3, E'4 of the same scale is provided from the encoder branch to the decoder branch of via a dedicated skip connection.

Therefore, the decoder only handles dimensionally-consistent n-dimensional feature maps (both the initial and enriched n-dimensional feature maps E'1, E'2, E'3, E'4, D1, D2, D3, D4 present the m spatial dimensions, possibly a batch dimension, and as n-thdimension said semantic depth), and it can only comprise standard convolution layers applying n-1-dimensional filters (bidimensional filters in the case of inputted tridimensional perfusion sequences). As a result, it is possible to have each enriched n-dimensional feature map D1, D2, D3, D4 incorporating the information from the initial n-dimensional feature maps of smaller or equal scale E'1, E'2, E'3, E'4.

In order to project the initial n+1-dimensional features maps E1, E2, E3, E4 into initial n-dimensional feature maps E'1, E'2, E'3, E'4, said skip connections may perform a temporal pooling operation (preferably a temporal max-pooling operation denoted maxt on the figure 3b, but other pooling such as average-pooling are possible).

Note that the smallest-scale initial n+1-dimensional features map E4 has already only one temporal channel, so that it is virtually already a n-dimensional feature map and the temporal dimension may be simply dropped, but alternatively any temporal pooling operation can still be performed (the result would be the same).

Like for any known decoder, at step (c) the smallest-scale enriched n-dimensional feature map D4 is generated from the smallest-scale initial n+1-dimensional feature map E4 (and preferably the smallest-scale initial n-dimensional feature map E'4 is directly taken as the smallest-scale enriched n-dimensional feature map D4, hence D4=E'4=E4 in the figure 3b as E4 has already only one temporal channel), and each other enriched n-dimensional feature map D1, D2, D3 is generated from the initial n-dimensional feature map E'1, E'2, E'3 of the same scale and a smaller-scale enriched n-dimensional feature map D2, D3, D4.

In other words, the decoder branch also consists of a plurality of successive convolution blocks but in opposite order, such that the first block produces the first enriched n-dimensional feature map D1 (from which the output image may be directly generated) from the second enriched n-dimensional feature map D2 and the first initial n-dimensional feature map E'1, after the second block produces the second enriched n-dimensional feature map D2 from the third enriched n-dimensional feature map D3 and the second initial n-dimensional feature map E'2, etc. The decoder branch is also preferably a fully convolutional CNN (direct succession of blocks of convolution layers and non-linear layers such as

10

ReLU).

Typically, the generation of an enriched n-dimensional map $D_i$ based on the corresponding initial n-dimensional map $E'_i$ and the smaller-scale enriched tridimensional map $D_{i+1}$ comprises rescaling of the enriched n-dimensional map $D_{i+1}$, typically doubling the scale (if there has been halving of scale in the encoder branch), i.e. up sampling of the enriched n-dimensional feature map for example with by a 2x2 convolution ("up-convolution") that halves or even divides by four the number of feature channels, then concatenation with the corresponding initial n-dimensional map $E'_i$ to keep or double again the number of channels, and from one block to another the number of filters of the convolution layers used (generally 5x5 convolutions) is again decreased (and preferably further halved).

For example, in the stU-Net of figure 3b:

- The smallest-scale enriched tridimensional map D4 (size 4x32x128) is directly the smallest-scale initial tridimensional map E'4 (since the latter already has the maximum semantic depth);
- The second smallest-scale enriched tridimensional map D3 (size 8x64x128) is generated from the second smallest-scale initial tridimensional map E'3 (size 8x64x64) provided via the skip connection and the smallest-scale enriched tridimensional map D4 (size 4x32x128), by up-convolution of D4 (doubling its size but halving its depth → resulting size (4x2)x(32x2)x(128/2)=8x64x64) then concatenation of initial tridimensional map E'3 with the up-convoluted D4 (resulting size 8x64x(64+64)=8x64x128), from which the convolution block generates D3 (number of convolution filters kept the same - but like in original decoder branch of U-Net it could have been halved - resulting size 8x64x128). Consequently, the information of the enriched tridimensional map D4 has been added to the initial tridimensional map E'3;

- The third smallest-scale enriched tridimensional map D2 (size 16x128x64) is generated from the third smallest-scale initial tridimensional map E'2 (size 16x128x32) provided via the skip connection and the second smallest-scale enriched tridimensional map D3 (size 8x64x128), by up-convolution of D3 (doubling its size but dividing by four its depth → resulting size (8x2)x(64x2)x(128/4)=16x128x32) then concatenation of initial tridimensional map E'2 with the up-convoluted D3 (resulting size 16x128x(32+32)=16x128x64), from which the convolution block generates D2 (number of convolution filters kept the same, resulting size 16x128x64). Consequently, the information of the enriched tridimensional map D3 has been added to the initial tridimensional map E'2;
- The largest-scale enriched tridimensional map D1 (size 32x256x1) is generated from the largest-scale initial tridimensional map E'1 (size 32x256x16) provided via the skip connection and the second largest-scale enriched tridimensional map D2 (size 16x128x64), by up-convolution of D2 (doubling its size but dividing by four its depth → resulting size (16x2)x(128x2)x(64/4)=32x256x16) then concatenation of initial tridimensional map E'1 with the up-convoluted D2 (resulting size 32x256x(16+16)=32x256x32), from which the convolution block generates D1 (a single 1x1 filter, resulting size 32x256x1). Consequently, the information of the enriched tridimensional map D2 has been added to the initial tridimensional map E'1.

Note that alternatively the skilled person could use any known architecture of 2D decoder, for instance the classical U-Net 2D encoder.

In a final step (d), at least one quantitative map of the inputted perfusion is generated from the largest-scale enriched n-dimensional feature map D1.

In particular, said quantitative maps can be directly extracted as channels of the largest-scale enriched n-dimensional feature map D1. Indeed, the at least one quantitative map only presents the two spatial dimensions.

In the example of figure 3b, we note that said largest-scale enriched n-dimensional feature map D1 has exactly the spatial size as the inputted patches (32x256) and one channel, i.e. it is directly a 32x256 quantitative map of a relevant parameter.

The final 1x1 convolution of the last convolution block acts as a calculator of the relevant parameter(s).

Note that step (d) may comprise rescaling (if the output spatial size does not perfectly match the input spatial size) and/or reconstruction of a complete image from the patches and/or the slices.

*Training method*

In a second aspect, there is proposed a training method, implemented by the data processor 11a of the first server 1a. Said method trains the stU-Net type CNN, for processing inputted perfusion sequence.

By training, it is meant the determination of the optimal values of parameters and weights or the CNN.

Note that the CNN used in the processing method is preferably trained according to the present training method, hence referred to at step (a0) in the figure 2. Note that alternatively the CNN may be directly taken "off the shelf" with preset values of parameters and weights.

Said training method is similar to the previously described processing method, but is iteratively performed on training perfusion sequences of the training database, i.e. the training perfusion sequence are associated with an expected quantitative map of the perfusion, for instance obtained using a reference algorithm (such as a known software, for example Verbena in the case of DSC-perfusion). Note that the generation of the expected quantitative maps may take as much time as necessary and the data processor 11a of the first server 1a is expected to have strong computational power (because it is independent of any clinical context, and only the quality of the result is important), so that known software are suitable.

In particular, the training method comprises, for each of the plurality of training perfusion sequences from the base of training perfusion sequences stored on the storage means 12a of the first server, an optional step (A) and then steps (B) et (D), respectively corresponding to steps (a) to (d) of the processing method.

In the optional step (A), the training perfusion sequence may be obtained (i.e. the expected quantitative maps generated) and possibly pre-processed (quality enhancement, patching, etc.).

In the step (B), the encoder branch extracts a plurality of initial n+1-dimensional features maps representative of the training perfusion sequence at different scales.

In the step (C), the decoder branch generates a plurality of enriched n-dimensional feature maps also representative of the training perfusion sequence at different scales, each enriched n-dimensional feature map incorporating the information from the initial n-dimensional feature maps of smaller or equal scale.

In a final step (D), at least one candidate quantitative map of the perfusion is generated from the largest-scale enriched n-dimensional feature map, so as to minimize a distance between said candidate quantitative map and the expected quantitative map of the perfusion.

In a preferred embodiment, the CNN is trained with the Adam optimizer, with or without a scheduler for setting the learning rate, with default hyper-parameters to minimize the L2 loss between the quantitative maps (or just the patches).

During training, patches can be sampled regularly from the original images with a stride of 8. At test time, patches can be sampled with a stride of 1 and all the predictions be assembled by averaging.

*Artificial degradation*

Data augmentation is known to play a central role in CNN training, and said training method preferably comprises generating at least one degraded version of at least one original training perfusion sequence of the training base, associating to said degraded version the expected quantitative map of the perfusion associated with the original training perfusion sequence, and enriching the training base with said degraded version (by adding it to the base). In other words, the degraded version associated with the original expected quantitative map is considered as a new training perfusion sequence in the training base. Note that there might be several degraded versions generated for the same original training perfusion sequence and/or degraded versions generated for several original training perfusion sequence.

While it is known to have degradation of the quality of training samples by image processing such as rotation, deformation, blurring, etc., the present method unprecedently proposes to simulate a low-dose contrast agent perfusion.

In other words, it is assumed that the original training perfusion sequence is associated to a contrast product dose (in particular a gadolinium-based contrast-agent), referred to as original dose (the dose used when the perfusion sequence had been acquired), and said degraded version of the original training perfusion sequence simulates a lower contrast product dose (than said dose associated to the original training perfusion sequence), referred to as degraded dose. For example, it can be chosen that said degraded dose is 50% of the original dose.

Such data augmentation is really advantageous to improve the performances of the CNN at low-doses (it is reminded that in first-pass perfusion methods such as DSC, the observed signal is proportional to the injected quantity of GBCA, thus a solution to have "less degraded" results is to increase this injected quantity), and therefore it ultimately allows a reduced use of contrast-product such as gadolinium, which is consistent with recent clinical guidelines that suggest, based on precautionary considerations, the use of the minimum dosage that achieves a sufficient contrast enhancement.

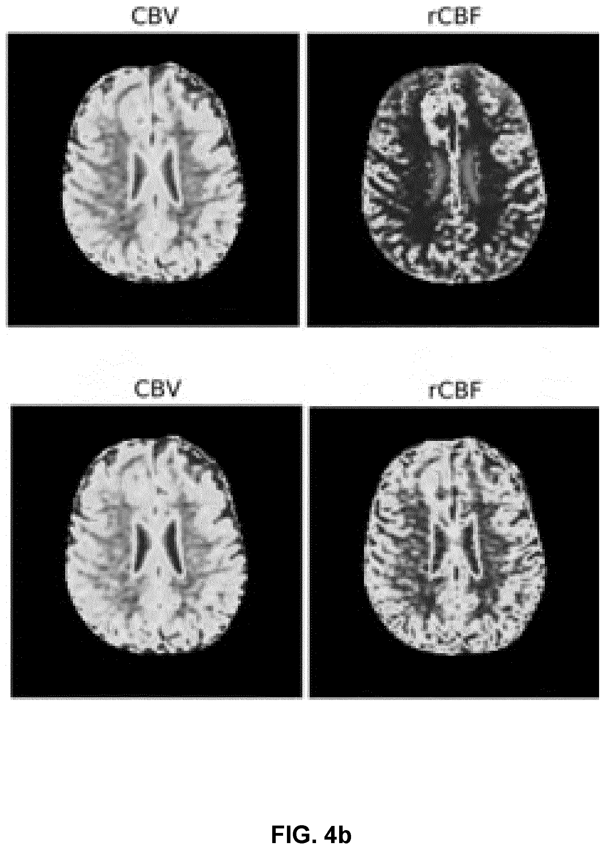

It is to be understood that "low-dose" training perfusion sequences could have been constructed by directly acquiring images using a reduced dose of contrast agent and obtaining the corresponding expected quantitative maps with the reference algorithm (such as the Verbena software). However, because of the low SNR inherent to such perfusion sequence, said quantitative maps would have been of low quality (see below the description of figure 4b). To the contrary, the present approach allows the reference algorithm to work with high SNR perfusion sequences for which it is known to be efficient, and to simulate from these high SNR perfusion sequences the low SNR perfusion sequence.

To rephrase again, in theory if the processing was perfect the resulting quantitative maps should be identical irrespective of the dose, that is why we can keep the high-quality quantitative maps obtained with high-SNR perfusion sequences as expected quantitative map for any lower-dose version.

According to the classical DSC-perfusion theory, the temporal signal S(t) in each voxel of the perfusion sequence varies linearly with the dose of contrast-product this voxel contains C(t) at any time t. In practice, only a noisy version of the true signal can be observed at a discrete set of regularly spaced time points *t,* typically every second, i.e. $t \in [\![1; T]\!]$:

$$S(t) = S(0) + \kappa.\,C(t) + \varepsilon(t),$$

where $\varepsilon(t) \sim \mathcal{N}(0, \sigma_\varepsilon^2)$

Introducing now the artificial dose reduction factor $0\% \leq d \leq 100\%$, the signal $S_d$ that would have been observed instead of the reference signal S if the dose had been reduced by a factor d, writes:

$$S_d(t) = S(0) + \kappa.\,C(t) \times d + \varepsilon(t),$$

As it will be shown, the degraded version of the original training perfusion sequence simulating a lower contrast product dose can advantageously be generated by calculating the degraded temporal signal $S_d(t)$, for each voxel of the original perfusion sequence, only as a function of the temporal signal $S(t)$ of said voxel and the dose reduction factor $d$.

Assuming that the temporal dynamics of the concentration time curve C(t) are slow with respect to the temporal sampling interval $\Delta$t (for instance 1s), we can write $\overline{C(t)} \approx \overline{C(t)}$ where the operator $\overline{(.)}$ denotes a local average, i.e. a "smoothed" value. Note that such a hypothesis is reasonable since it actually corresponds to a desired behavior when the operator selects the sampling interval $\Delta$t.

Since the noise distribution is assumed independent of time, we can finally write $\overline{\varepsilon(t)} \approx 0$ and therefore $\varepsilon(t) \approx S(t) - \overline{S}$ $\overline{(t)}$.

This leads to:

$$S_d(t) \approx S(t) - (1 - d) \cdot [\overline{S(t)} - S(0)]$$

This synthetic signal $S_d$ can thus be easily computed for any reference signal S or artificial dose reduction factor d.

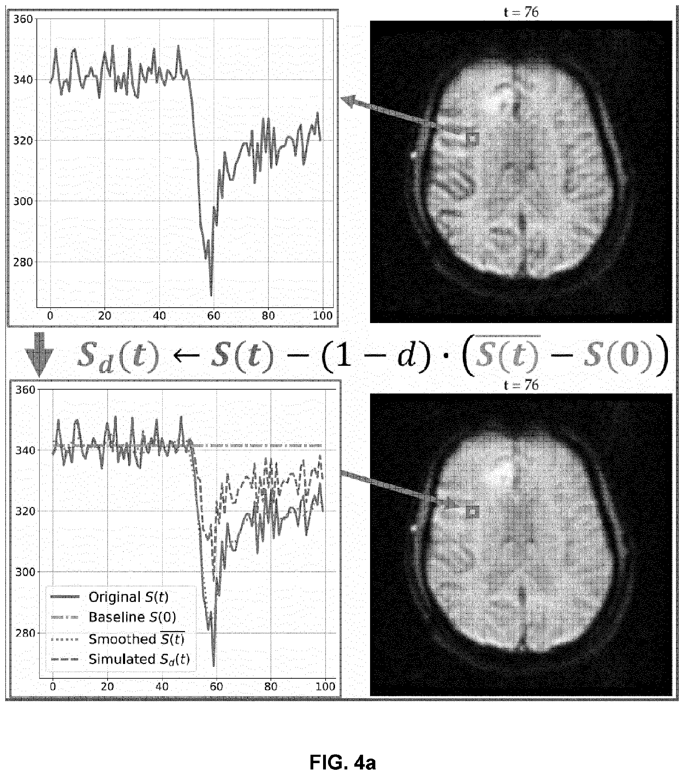

**Figure 4a** displays an example of the proposed approach, which effectively allows the manipulation of the SNR of reference perfusion sequences in a realistic and theoretically-consistent manner: Based on an original perfusion sequence (top right image), a synthetic 50%-dose sequence is simulated, i.e. the "degraded version" (bottom right image). The temporal signal contained in each voxel S(t) (solid line on both left figures) is transformed into a degraded temporal signal $S_d(t)$ (dotted --- line on the bottom left figure). The ····· and -·-· dotted lines on the bottom left figure respectively correspond to a local average S(t) and the baseline signal S(0).

**Figure 4b** represent on top two expected quantitative maps (CBV and rCBF) for the original perfusion sequence of figure 4a (which are thus also expected quantitative maps that are to be associated to the artificially degraded version of the original perfusion sequence of figure 4a), and on bottom the two quantitative maps that would have been obtained for said degraded version of figure 4a if this degraded version was directly used as a training perfusion sequence: we see that many information would have been lost, especially for the rCBF (note the anomaly on the front part of the brain that is barely visible on the bottom map).

In other words, thanks to the present approach we are able to train the CNN to generate the high-quality quantitative maps directly from the low-SNR perfusion sequences obtained with a large range of contrast product doses and in particular a low contrast product dose.

*Tests*

The predictive performance of the stU-Net can be evaluated and compared to known solutions on a public data set containing 49 patients with glioma (see the document KM Schmainda, MA Prah, JM Connelly, and SD Rand, "Glioma dsc-mri perfusion data with standard imaging and rois," The Cancer Imaging Archive. http://doi.org/10.7937 K, vol. 9, 2016).

Raw brain DSC-perfusion sequences are readily available for all subjects, along with individual segmentation maps of a large vessel from which the AIF can be estimated. Expected quantitative CBV maps are generated using the SVD pipeline of Verbena v4.0, freely distributed as part of the FSL v6.0 software suite.

Corresponding pairs of DSC perfusion sequences and CBV maps are individually cropped on square prisms tightly fitted on the brain voxels, and each slice is then resampled to a 256x256 size. The DSC and CBV signals are then standardized using the brain content statistics, and extreme values are clipped. All DSC frames are normalized similarly, according to the signal statistics of the first frame only.

The results are shown in the table 1 herebelow.

| | | | Verbena | Hess | stU-Net |
|---|---|---|---|---|---|
| full-dose | L2 | $(10^{-2})$ | (n/a) | 38.71 (15.06) | **8.38** (3.52) |
| | t-L2 | $(10^{-2})$ | (n/a) | 26.30 (67.29) | **5.93** (6.44) |
| | L1 | $(10^{-2})$ | (n/a) | 34.01 (5.49) | **17.58** (4.05) |
| | t-L1 | $(10^{-2})$ | (n/a) | 26.89 (21.51) | **16.52** (9.37) |
| | SSIM | (%) | (n/a) | 63.16 (5.71) | **82.08** (5.02) |
| ½-dose | L2 | $(10^{-2})$ | 15.97 (3.70) | 39.00 (15.95) | **8.86** (2.98) |
| | t-L2 | $(10^{-2})$ | 11.24 (15.50) | 28.09 (72.44) | **3.95** (4.17) |
| | L1 | $(10^{-2})$ | 22.69 (3.70) | 33.87 (6.04) | **18.97** (3.76) |
| | t-L1 | $(10^{-2})$ | 20.62 (14.59) | 27.31 (22.47) | **13.96** (7.07) |
| | SSIM | (%) | 77.90 (4.22) | 63.67 (5.47) | **79.19** (5.17) |
| ¼-dose | L2 | $(10^{-2})$ | 70.90 (74.47) | 39.82 (15.86) | **9.68** (2.72) |
| | t-L2 | $(10^{-2})$ | 38.67 (43.45) | 30.06 (70.35) | **6.26** (7.48) |
| | L1 | $(10^{-2})$ | 46.16 (19.11) | 34.72 (6.09) | **18.22** (2.35) |
| | t-L1 | $(10^{-2})$ | 41.66 (24.32) | 30.07 (22.32) | **17.53** (10.89) |
| | SSIM | (%) | 59.08 (6.57) | 62.78 (5.37) | **81.36** (3.91) |

Table 1

Table 1 reports quantitative results, in terms of the L1, tumor-averaged L1 (t-L1), L2, tumor-averaged L2 (t-L2) and structural similarity (SSIM) performance metrics (means and standard deviations) achieved in the full-dose, half-dose and quarter-dose scenarios by the SVD pipeline of the Verbena software, the state-of-the art method of Hess, and the present stU-Net approach. Verbena generated the reference full-dose CBV maps, and therefore cannot be evaluated in this scenario (indicated as n/a in the Table).

Metrics are only computed on the test cases. The best metric of each row is indicated in bold, the second best is underlined.

In the quarter-dose regime, Verbena becomes outperformed by Hess, as all its performance metric drop sharply. However, in all scenarios and for all metrics, the stU-Net largely outperforms on average both the method of Hess and Verbena.

*Computer program product*

In a third aspect, the invention provides a computer program product comprising code instructions to execute a method (particularly on the data processor 11a, 11b of the first or second server 1a, 1b) according to the second aspect of the invention for training a convolution neural network CNN, or a method according to the first aspect of the invention for processing an inputted perfusion sequence.

**Claims**

1.    A method for processing an inputted perfusion sequence representing a stack of a plurality of images successively depicting passage of a fluid within a body part over time and presenting n≥3 dimensions including at least two spatial dimensions and one temporal dimension, by means of a convolutional neural network, CNN, the method comprising the implementation, by a data processor (11b) of a second server (1b), of steps of:

(b) extracting, using an encoder branch of the CNN, a plurality of initial n+1-dimensional features maps representative of the inputted perfusion sequence at different scales, said CNN further comprising a decoder branch and skip connections between the encoder branch and the decoder branch, wherein each skip connection projects one of the initial n+1-dimensional features maps into one of a plurality of initial n-dimensional feature maps;

(c) generating, using said decoder branch of the CNN, a plurality of enriched n-dimensional feature maps also representative of the inputted perfusion sequence at different scales, each enriched n-dimensional feature map incorporating the information from the initial n-dimensional feature maps of smaller or equal scale;

(d) generating at least one quantitative map of a parameter characterizing perfusion over the body part from the largest-scale enriched n-dimensional feature maps.

2. A method according to claim 1, wherein, for each enriched n-dimensional feature map, an initial n-dimensional feature map of the same scale is provided from the encoder branch to the decoder branch - via a dedicated skip connection.

3. The method according to one of claims 1 and 2, wherein, at step (c), the smallest-scale enriched n-dimensional feature map is generated from the smallest-scale initial n+1-dimensional feature map, and each other enriched n-dimensional feature map is generated from the initial n-dimensional feature map of the same scale and a smaller-scale enriched n-dimensional feature map.

4. The method according to one of claims 1 to 3, comprising a previous step (a) of obtaining the perfusion sequence by stacking a plurality of successive images of a perfusion.

5. The method according to claim 4, wherein said successive images are acquired by a medical imaging device (10) connected to the second server (1a).

6. The method according to claim 5, wherein said medical imaging device is a Magnetic Resonance Imaging, MRI, scanner, and the perfusion sequence is Dynamic susceptibility Contrast, DSC, or a Dynamic Contrast Enhanced, DCE, perfusion sequence; said quantitative map being preferably a map of parameter chosen among a cerebral blood volume, CBV, a cerebral blood flow, CBF, a mean transit time, MTT, and a k-trans.

7. The method according to one of claims 4 to 6, wherein step (a) comprises extracting patches of a predetermined size from the perfusion sequence, steps (b) to (d) being performed for each extracted patch.

8. The method according to one of claims 1 to 7, wherein said CNN is fully convolutional.

9. The method according to one of claims 1 to 8, wherein the at least one quantitative map only presents the spatial dimensions of the inputted perfusion sequence; the initial n+1-dimensional feature maps present said spatial and temporal dimensional dimensions and as n+1-th dimension a semantic depth; and said initial and enriched n-dimensional feature maps present said spatial dimensions and as n-th dimension said semantic depth; the number of said spatial dimensions being in particular n-1, and preferably n=3.

10. The method according to claim 9, wherein said skip connections perform a temporal pooling operation.

11. A method for training a convolution neural network, CNN, for processing an inputted perfusion sequence representing a stack of a plurality of images successively depicting passage of a fluid within a body part over time and presenting $n \geq 3$ dimensions including at least two spatial dimensions and one temporal dimension;

the method comprising the implementation, by a data processor (11a) of a first server (1a), for each of a plurality of training perfusion sequence from a base of training perfusion sequences each associated to an expected quantitative map of a parameter characterizing perfusion over the body part, the method comprising steps of:

(B) extracting, using an encoder branch of the CNN, a plurality of initial n+1-dimensional features maps representative of the training perfusion sequence at different scales, $n \geq 3$, said CNN further comprising a decoder branch and skip connections between the encoder branch and the decoder branch, each skip connection projecting one of the initial n+1-dimensional features maps into one of a plurality of initial n-dimensional feature maps;

(C) generating, using said decoder branch of the CNN, a plurality of enriched n-dimensional feature maps also representative of the training perfusion sequence at different scales, each enriched n-dimensional feature map incorporating the information from the initial n-dimensional feature maps of smaller or equal scale;

(D) generating at least one candidate quantitative map of the parameter characterizing perfusion over the body part from the largest-scale enriched n-dimensional feature map, and minimizing a distance with the expected quantitative map.

12. The method according to claim 11, previously comprising generating at least one degraded version of at least one original training perfusion sequence of the training base, associating to said degraded version the expected quantitative map of the perfusion associated with the original training perfusion sequence, and enriching the training base by adding said degraded version.

13. The method according to claim 12, wherein said original training perfusion sequence is associated to a contrast product dose, said degraded version of the original training perfusion sequence simulating a lower contrast product dose.

14. The method according to claim 13, wherein the degraded version of the original training perfusion sequence simulating a lower contrast product dose is generated by calculating, for each voxel of the original training perfusion sequence, from the temporal signal $S(t)$ of said voxel a degraded temporal signal $S_d(t)$ using the formula $S_d(t) = S(t) - (1 - d) \cdot [\overline{S(t)} - S(0)]$, wherein $\overline{S(t)}$ is a local average of the temporal signal $S(t)$, and $d$ is a dose reduction factor.

15. Computer program product comprising code instructions to execute a method according to one of claims 1 to 14 for training a convolution neural network CNN, or for processing an inputted perfusion sequence, when said program is executed on a computer.

**Patentansprüche**

1. Verfahren zum Verarbeiten einer eingegebenen Perfusionssequenz, die einen Stapel einer Vielzahl von Bildern repräsentiert, die nacheinander den Durchgang eines Fluids in einem Körperteil im Laufe der Zeit darstellen und n≥3 Dimensionen aufweisen, einschließlich mindestens zweier räumlicher Dimensionen und einer zeitlichen Dimension, mittels eines "Convolutional Neural Network", CNN, wobei das Verfahren die Implementierung, durch einen Datenprozessor (11b) eines zweiten Servers (1b), der folgenden Schritte umfasst:

(b) Extrahieren, unter Verwendung eines Kodiererzweigs des CNN, einer Vielzahl von anfänglichen n+1-dimensionalen Merkmalskarten, die für die eingegebene Perfusionssequenz in verschiedenen Maßstäben repräsentativ sind, wobei das CNN ferner einen Dekodiererzweig und Skip-Verbindungen zwischen dem Kodiererzweig und dem Dekodiererzweig umfasst, wobei jede Skip-Verbindung eine der anfänglichen n+1-dimensionalen Merkmalskarten in eine aus einer Vielzahl von anfänglichen n-dimensionalen Merkmalskarten projiziert;
(c) Erzeugen, unter Verwendung des Dekodiererzweigs des CNN, einer Vielzahl von angereicherten n-dimensionalen Merkmalskarten, die ebenfalls repräsentativ für die eingegebene Perfusionssequenz in verschiedenen Maßstäben sind, wobei jede angereicherte n-dimensionale Merkmalskarte die Informationen aus den anfänglichen n-dimensionalen Merkmalskarten mit kleinerem oder gleichem Maßstab einbezieht;
(d) Erzeugen mindestens einer quantitativen Karte eines Parameters, der die Perfusion über den Körperteil charakterisiert, aus den am größten skalierten angereicherten n-dimensionalen Merkmalskarten.

2. Verfahren nach Anspruch 1, wobei für jede angereicherte n-dimensionale Merkmalskarte eine anfängliche n-dimensionale Merkmalskarte des gleichen Maßstabs vom Kodiererzweig zum Dekodiererzweig - über eine spezielle Skip-Verbindung - bereitgestellt wird.

3. Verfahren nach einem der Ansprüche 1 und 2, wobei in Schritt (c) die am kleinsten skalierte angereicherte n-dimensionale Merkmalskarte aus der am kleinsten skalierten anfänglichen n+1-dimensionalen Merkmalskarte erzeugt wird, und jede andere angereicherte n-dimensionale Merkmalskarte aus der anfänglichen n-dimensionalen Merkmalskarte desselben Maßstabs und einer kleiner skalierten angereicherten n-dimensionalen Merkmalskarte erzeugt wird.

4. Verfahren nach einem der Ansprüche 1 bis 3, umfassend einen vorhergehenden Schritt (a) des Erhaltens der Perfusionssequenz durch Stapeln einer Vielzahl von aufeinanderfolgenden Bildern einer Perfusion.

5. Verfahren nach Anspruch 4, wobei die aufeinanderfolgenden Bilder von einer medizinischen Bildgebungsvorrichtung (10) erfasst werden, die mit dem zweiten Server (1a) verbunden ist.

6.  Verfahren nach Anspruch 5, wobei die medizinische Bildgebungsvorrichtung ein Magnetresonanztomographie-, MRT-, Scanner ist und die Perfusionssequenz eine "Dynamic Susceptibility Contrast"-, DSC-, oder eine "Dynamic Contrast Enhanced"-, DCE-, Perfusionssequenz ist; wobei die quantitative Karte vorzugsweise eine Karte eines Parameters ist, der aus einem zerebralen Blutvolumen, CBV, einem zerebralen Blutfluss, CBF, einer mittleren Transitzeit, MTT, und einem k-trans ausgewählt ist.

7.  Verfahren nach einem der Ansprüche 4 bis 6, wobei Schritt (a) das Extrahieren von Patches einer vorbestimmten Größe aus der Perfusionssequenz umfasst und die Schritte (b) bis (d) für jeden extrahierten Patch durchgeführt werden.

8.  Verfahren nach einem der Ansprüche 1 bis 7, wobei das CNN "fully convolutional" ist.

9.  Verfahren nach einem der Ansprüche 1 bis 8, wobei die mindestens eine quantitative Karte nur die räumlichen Dimensionen der eingegebenen Perfusionssequenz aufweist; die anfänglichen n+1-dimensionalen Merkmalskarten die räumlichen und die zeitlichen Dimensionen und als n+1-te Dimension eine semantische Tiefe aufweisen; und die anfänglichen und die angereicherten n-dimensionalen Merkmalskarten die räumlichen Dimensionen und als n-te Dimension die semantische Tiefe aufweisen; wobei die Anzahl der räumlichen Dimensionen insbesondere n-1 und vorzugsweise n=3 ist.

10. Verfahren nach Anspruch 9, wobei die Skip-Verbindungen eine zeitliche Pooling-Operation durchführen.

11. Verfahren zum Trainieren "Convolutional Neural Network", CNN, zum Verarbeiten einer eingegebenen Perfusionssequenz, die einen Stapel aus einer Vielzahl von Bildern repräsentiert, die nacheinander den Durchgang eines Fluids in einem Körperteil im Laufe der Zeit darstellen und n≥3 Dimensionen aufweisen, einschließlich mindestens zweier räumlicher Dimensionen und einer zeitlichen Dimension;
    wobei das Verfahren die Implementierung, durch einen Datenprozessor (11a) eines ersten Servers (1a), für jede einer Vielzahl von Trainingsperfusionssequenzen aus einer Basis von Trainingsperfusionssequenzen, die jeweils einer erwarteten quantitativen Karte eines Parameters zugeordnet sind, der die Perfusion über den Körperteil charakterisiert, der folgenden Schritte umfasst:

    (B) Extrahieren, unter Verwendung eines Kodiererzweigs des CNN, einer Vielzahl von anfänglichen n+1-dimensionalen Merkmalskarten, die für die Trainingsperfusionssequenz in verschiedenen Maßstäben, n≥3, repräsentativ sind, wobei das CNN ferner einen Dekodiererzweig und Skip-Verbindungen zwischen dem Kodiererzweig und dem Dekodiererzweig umfasst, wobei jede Skip-Verbindung eine der anfänglichen n+1-dimensionalen Merkmalskarten in eine aus einer Vielzahl von anfänglichen n-dimensionalen Merkmalskarten projiziert;
    (C) Erzeugen, unter Verwendung des Dekodiererzweigs des CNN, einer Vielzahl von angereicherten n-dimensionalen Merkmalskarten, die ebenfalls repräsentativ für die Trainingsperfusionssequenz in verschiedenen Maßstäben sind, wobei jede angereicherte n-dimensionale Merkmalskarte die Informationen aus den anfänglichen n-dimensionalen Merkmalskarten mit kleinerem oder gleichem Maßstab einbezieht;
    (D) Erzeugen von mindestens einer in Frage kommenden quantitativen Karte des Parameters, der die Perfusion über den Körperteil charakterisiert, aus der am größten skalierten angereicherten n-dimensionalen Merkmalskarte, und Minimieren eines Abstands zur erwarteten quantitativen Karte.

12. Verfahren nach Anspruch 11, zuvor umfassend das Erzeugen mindestens einer verschlechterten Version mindestens einer ursprünglichen Trainingsperfusionssequenz der Trainingsbasis, das Zuordnen der erwarteten quantitativen Karte der Perfusion, die der ursprünglichen Trainingsperfusionssequenz zugeordnet ist, zur verschlechterten Version und das Anreichern der Trainingsbasis durch Hinzufügen der verschlechterten Version.

13. Verfahren nach Anspruch 12, wobei die ursprüngliche Trainingsperfusionssequenz einer Kontrastmitteldosis zugeordnet ist und die verschlechterte Version der ursprünglichen Trainingsperfusionssequenz eine niedrigere Kontrastmitteldosis simuliert.

14. Verfahren nach Anspruch 13, wobei die verschlechterte Version der ursprünglichen Trainingsperfusionssequenz, die eine niedrigere Kontrastmitteldosis simuliert, erzeugt wird, indem für jedes Voxel der ursprünglichen Trainingsperfusionssequenz aus dem zeitlichen Signal S(t) des Voxels ein verschlechtertes zeitliches Signal $S_d(t)$ unter Verwendung der Formel $S_d(t) = S(t) - (1 - d) \cdot [\overline{S(t)} - S(0)]$ berechnet wird, wobei $\overline{S(t)}$ ein lokaler Mittelwert des zeitlichen Signals $S(t)$ ist und $d$ ein Dosisreduktionsfaktor ist.

15. Computerprogrammprodukt, umfassend Codebefehle zum Ausführen eines Verfahrens nach einem der Ansprüche 1 bis 14 zum Trainieren eines "Convolutional Neural Network", CNN, oder zum Verarbeiten einer eingegebenen Perfusionssequenz, wenn das Programm auf einem Computer ausgeführt wird.

**Revendications**

1. Procédé de traitement d'une séquence de perfusion fournie en entrée représentant un empilement d'une pluralité d'images illustrant successivement le passage d'un fluide à l'intérieur d'une partie corporelle au cours du temps et présentant n≥3 dimensions dont au moins deux dimensions spatiales et une dimension temporelle, au moyen d'un réseau de neurones convolutif, CNN, le procédé comprenant la mise en oeuvre, par un processeur de données (11b) d'un second serveur (1b), des étapes consistant à :

   (b) extraire, au moyen d'une branche codeuse du CNN, une pluralité de cartes initiales de caractéristiques n+1-dimensionnelles représentant la séquence de perfusion fournie en entrée à différentes échelles, ledit CNN comprenant en outre une branche décodeuse et des connexions de saut entre la branche codeuse et la branche décodeuse, chaque connexion de saut projetant l'une des cartes initiales de caractéristiques n+1-dimensionnelles vers l'une d'une pluralité de cartes initiales de caractéristiques n-dimensionnelles ;
   (c) générer, au moyen de ladite branche décodeuse du CNN, une pluralité de cartes enrichies de caractéristiques n-dimensionnelles représentant également la séquence de perfusion fournie en entrée à différentes échelles, chaque carte enrichie de caractéristiques n-dimensionnelles incorporant les informations provenant des cartes initiales de caractéristiques n-dimensionnelles d'échelle inférieure ou égale ;
   (d) générer au moins une carte quantitative d'un paramètre caractérisant la perfusion sur la partie corporelle à partir des cartes enrichies de caractéristiques n-dimensionnelles à l'échelle la plus grande.

2. Procédé selon la revendication 1, dans lequel, pour chaque carte enrichie de caractéristiques n-dimensionnelles, une carte initiale de caractéristiques n-dimensionnelles à la même échelle est fournie de la branche codeuse à la branche décodeuse - par le biais d'une connexion de saut dédiée.

3. Procédé selon l'une des revendications 1 et 2, dans lequel, à l'étape (c), la carte enrichie de caractéristiques n-dimensionnelles à la plus petite échelle est générée à partir de la carte initiale de caractéristiques n+1-dimensionnelles à la plus petite échelle, et chaque autre carte enrichie de caractéristiques n-dimensionnelles est générée à partir de la carte initiale de caractéristiques n-dimensionnelles de même échelle et d'une carte enrichie de caractéristiques n-dimensionnelles d'échelle inférieure.

4. Procédé selon l'une des revendications 1 à 3, comprenant une étape préalable (a) consistant à obtenir la séquence de perfusion par empilement d'une pluralité d'images successives d'une perfusion.

5. Procédé selon la revendication 4, dans lequel lesdites images successives sont acquises par un dispositif d'imagerie médicale (10) connecté au second serveur (1a).

6. Procédé selon la revendication 5, dans lequel ledit dispositif d'imagerie médicale est un dispositif de balayage d'imagerie par Résonance Magnétique, IRM, et la séquence de perfusion est une séquence de perfusion à Contraste de susceptibilité Dynamique, DSC, ou une séquence de perfusion à Amélioration de Contraste Dynamique, DCE ; ladite carte quantitative étant de préférence une carte de paramètres choisis parmi un volume sanguin cérébral, CBV, un débit sanguin cérébral, CBF, un temps de transit moyen, MTT, et un k-trans.

7. Procédé selon l'une des revendications 4 à 6, dans lequel l'étape (a) consiste à extraire des plages de taille prédéterminée à partir de la séquence de perfusion, les étapes (b) à (d) étant mises en œuvre pour chaque plage extraite.

8. Procédé selon l'une des revendications 1 à 7, dans lequel ledit CNN est entièrement convolutif.

9. Procédé selon l'une des revendications 1 à 8, dans lequel l'au moins une carte quantitative présente uniquement les dimensions spatiales de la séquence de perfusion fournie en entrée ; les cartes initiales de caractéristiques n+1-dimensionnelles présentent lesdites dimensions spatiales et temporelles et, en tant que n+1-ième dimension, une profondeur sémantique ; et lesdites cartes initiales de caractéristiques n-dimensionnelles et enrichies présentent lesdites dimensions spatiales et, en tant que n-ième dimension, ladite profondeur sémantique ; le nombre desdites dimensions spatiales étant en particulier égal à n-1, et de préférence n=3.

10. Procédé selon la revendication 9, dans lequel lesdites connexions de saut mettent en œuvre une opération de regroupement temporel.

11. Procédé d'entraînement d'un réseau de neurones convolutif, CNN, pour le traitement d'une séquence de perfusion fournie en entrée représentant un empilement d'une pluralité d'images illustrant successivement le passage d'un fluide à l'intérieur d'une partie corporelle au cours du temps et présentant n≥3 dimensions dont au moins deux dimensions spatiales et une dimension temporelle ;
le procédé comprenant la mise en oeuvre, par un processeur de données (11a) d'un premier serveur (1a), pour chacune d'une pluralité de séquence de perfusion d'apprentissage provenant d'une base de séquences de perfusion d'apprentissage associées chacune à une carte quantitative attendue d'un paramètre caractérisant la perfusion sur la partie corporelle, le procédé comprenant les étapes consistant à :

(B) extraire, au moyen d'une branche codeuse du CNN, une pluralité de cartes initiales de caractéristiques n+1-dimensionnelles représentant la séquence de perfusion d'apprentissage à différentes échelles, n≥3, ledit CNN comprenant en outre une branche décodeuse et des connexions de saut entre la branche codeuse et la branche décodeuse, chaque connexion de saut projetant l'une des cartes initiales de caractéristiques n+1-dimensionnelles vers l'une d'une pluralité de cartes initiales de caractéristiques n-dimensionnelles ;
(C) générer, au moyen de ladite branche décodeuse du CNN, une pluralité de cartes enrichies de caractéristiques n-dimensionnelles représentant également la séquence de perfusion d'apprentissage à différentes échelles, chaque carte enrichie de caractéristiques n-dimensionnelles incorporant les informations provenant des cartes initiales de caractéristiques n-dimensionnelles d'échelle inférieure ou égale ;
(D) générer au moins une carte quantitative candidate du paramètre caractérisant la perfusion sur la partie corporelle à partir de la carte enrichie de caractéristiques n-dimensionnelles à l'échelle la plus grande, et réduire au minimum une distance par rapport à la carte quantitative attendue.

12. Procédé selon la revendication 11, consistant préalablement à générer au moins une version dégradée d'au moins une séquence de perfusion d'apprentissage originale de la base d'apprentissage, associer à ladite version dégradée la carte quantitative attendue de la perfusion associée à la séquence de perfusion d'apprentissage originale, et enrichir la base d'apprentissage par ajout de ladite version dégradée.

13. Procédé selon la revendication 12, dans lequel ladite séquence de perfusion d'apprentissage originale est associée à une dose de produit de contraste, ladite version dégradée de la séquence de perfusion d'apprentissage originale simulant une dose inférieure de produit de contraste.

14. Procédé selon la revendication 13, dans lequel la version dégradée de la séquence de perfusion d'apprentissage originale simulant une dose inférieure de produit de contraste est générée par le calcul, pour chaque voxel de la séquence de perfusion d'apprentissage originale, à partir du signal temporel $S(t)$ dudit voxel, un signal temporel dégradé $S_d(t)$ au moyen de la formule $S_d(t) = S(t) - (1 - d) - [\overline{S(t)} - S(0)]$, où $\overline{S(t)}$ est une moyenne locale du signal temporel $S(t)$, et d est un facteur de réduction de la dose.

15. Produit programme d'ordinateur comprenant des instructions de code permettant d'exécuter un procédé selon l'une des revendications 1 à 14 pour l'apprentissage d'un réseau de neurones convolutif, CNN, ou pour le traitement d'une séquence de perfusion fournie en entrée, lorsque ledit programme est exécuté sur un ordinateur.

**FIG. 1**

Training Base

(a0) Training the CNN

(a) Obtaining the perfusion sequence

(b) Extracting initial features maps

(c) Generating enriched features maps

(d) Generating quantitative maps

**FIG. 2**

FIG. 3a

EP 4 264 308 B1

# FIG. 3b

1 channel
32x256x120

16 channels
32x256x120

E1

3x3x9

2x2x4

32 channels
16x128x30

E2

3x3x7

2x2x4

64 channels
8x64x7

E3

3x3x7

2x2x4

128 channels
4x32x1

3x3x1

128 channels
4x32

E4=E'4=D4

2+1D encoder

max_t

max_t

max_t

2x2

E'3

5x5

D3

128 channels
8x64

2x2

E'2

5x5

D2

64 channels
16x128

2x2

32 channels
32x256

E'1

1x1

1 channel
32x256

D1

2D decoder

EP 4 264 308 B1

$$S_d(t) \leftarrow S(t) - (1-d) \cdot \left( \overline{S(t)} - S(0) \right)$$

FIG. 4a

CBV

rCBF

CBV

rCBF

**FIG. 4b**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2017192629 A

- CN 110223285 A

**Non-patent literature cited in the description**

- Synthetic perfusion maps: imaging perfusion deficits in dsc-mri with deep learning. **ANDREAS HESS** ; **RAPHAEL MEIER** ; **JOHANNES KAESMACHER** ; **SIMON JUNG** ; **FABIEN SCALZO** ; **DAVID LIEBES-KIND** ; **ROLAND WIEST** ; **RICHARD MCKINLEY**. International MICCAI brain lesion workshop.. Springer, 2018, 447-455
- Synthetic perfusion maps: imaging perfusion deficits in dsc-mri with deep learning. **ANDREAS HESS** ; **RAPHAEL MEIER** ; **JOHANNES KAESMACHER** ; **SIMON JUNG** ; **FABIEN SCALZO** ; **DAVID LIEBES-KIND** ; **ROLAND WIEST** ; **RICHARD MCKINLEY**. International MICCAI brain lesion workshop. Springer, 2018, 447-455

- U-net: Convolutional networks for biomedical image segmentation. **OLAF RONNEBERGER** ; **PHILIPP FISCHER** ; **THOMAS BROX**. International Conference on Medical image computing and computer-assisted intervention. Springer, 2015, 234-241
- V-net: Fully convolutional neural networks for volumetric medical image segmentation. **FAUSTO MILLETARI** ; **NASSIR NAVAB** ; **SEYED-AHMAD AHMADI**. 2016 fourth international conference on 3D vision (3DV). IEEE, 2016, 565-571
- **KM SCHMAINDA** ; **MA PRAH** ; **JM CONNELLY** ; **SD RAND**. Glioma dsc-mri perfusion data with standard imaging and rois. *The Cancer Imaging Archive*, 2016, vol. 9, http://doi.org/10.7937 K